(19) Europäisches Patentamt
European Patent Office
Office européen des brevets

(11) Publication number: 0 325 225 A2

# (12) EUROPEAN PATENT APPLICATION

(21) Application number: 89100788.2

(22) Date of filing: 18.01.89

(51) Int. Cl.[4]: C07K 3/20 , C12N 9/64 , C12Q 1/56

(30) Priority: 18.01.88 CS 324/88
12.02.88 CS 869/88
28.04.88 CS 2890/88

(43) Date of publication of application: 26.07.89 Bulletin 89/30

(84) Designated Contracting States: CH DE FR GB IT LI

(71) Applicant: TESSEK SDRUZENI PRAHA
Krizovnická 3
CS-110 00 Praha 1(CS)

(72) Inventor: Rybák, Miroslav, Dipl.Ing.
Nesporova 573
Praha 4(CS)
Inventor: Kasafirek, Evzen, Dipl.-Ing.
Lomena 27
Praha 6(CS)
Inventor: Housková, Jitka, Dipl.-Ing.
Tyrsova 480
Letovice(CS)
Inventor: Losticky, Cyril, RNdr.
Podskalská 18
Praha 2(CS)
Inventor: Ulrych, Stanislav, RNdr.
Kovpakova 2
Praha 2(CS)
Inventor: Sedlmaier, Oldrich
Zborovská 14
Praha 5(CS)
Inventor: Roubalová, Alena
Holandská 23
Praha 10(CS)

(74) Representative: Patentanwälte Beetz sen. - Beetz jun. Timpe - Siegfried - Schmitt-Fumian- Mayr
Steinsdorfstrasse 10
D-8000 München 22(DE)

## (54) Concentrates of coagulation factors II, VII, IX and/or X, their preparation and use, and method for the determination of coagulation factor VII.

(57) The invention relates to concentrates of coagulation factors II, VII, IX and/or X and the method of their preparation by selective sorption on an equilibrated carrier on the basis of poly(DEAE-hydroxyethylmethacrylate) or poly(DEAE-hydroxyethylacrylate) and following selective desorption.

The concentrates of coagulation factors II and X can be used for the determination of coagulation factor VII and for the detection or determination of the extent of mammalian inflammatory diseases.

EP 0 325 225 A2

# Concentrates of coagulation factors II, VII, IX and/or X, their preparation and use,and method for the determination of coagulation factor VII

The invention relates to concentrates of coagulation factors from human and animal blood plasma, or from their fractions, the so-called "prothrombin complex", a group of coagulation factors dependent on vitamin K and known as coagulation factors II, VII, IX and X, respectively, the method of their preparation and their use, particularly for the determination of factor VII.

These coagulation factors in the form of more or less pure concentrates represent an important material for the preparation of parenteral preparates for the clinical and analytical praxis and the laboratory test and diagnosis of coagulation disorders, or they may serve as markers of certain diseases. The preparates are also used by patients suffering from a hereditary or acquired deficiency in blood coagulation, damage of liver parenchyme, vitamin K deficiency, an acute hemorrhagy, etc. , or for the pre-surgery prophylaxis of bleeding.

The established method of preparation of these coagulation factor concentrates using sorbents based on a polysaccharide matrix (for example diethylaminoethyl (DEAE) derivatives of polysaccharides) has some disadvantages: The polysaccharide matrix is highly susceptible to microbial contamination, which causes changes in the physical and chemical properties of the sorbent. Polysaccharide sorbent particles are only poorly resistant against changes of environmental conditions, e.g. temperature, pressure, salt concentration etc., which, accordingly may lead to a change of the sorbent and sorbent bed volume with all the undesirable consequences resulting therefrom.

It is the object of the present invention to provide concentrates of human or animal coagulation factors II, VII, IX and/or X, a method of their preparation and their use, and a method and kits for the qualitative detection and the quantitative determination of coagulation factor VII, particularly in biochemical materials, such as blood plasma, plasma fractions and preparations, and in body fluids and secretions, such as milk, urine etc.

The above object is achieved according to the claims. The dependent claims relate to preferred embodiments.

The concentrates and the method according to the present invention are based on the surprising finding that poly(diethylaminoethyl-hydroxyethylacrylate) and poly(diethylaminoethyl-hydroxyethylmethacrylate) sorbents are suited for the very complex separation and fractionation of blood coagulation factors II, VII, IX and X.

The concentrates of the blood coagulation factors II, VII, IX and/or X according to the present invention are obtainable by contacting a biological material containing these factors with a sorbent on the basis of poly(diethylaminoethyl-hydroxyethylacrylate) and/or poly(diethylaminoethyl-hydroxyethylmethacrylate) and desorption with a buffer solution of pH 7.2 to 7.6 having a saline concentration of 0.3 to 2.0 mol/l.

They are particularly obtainable by the following method:

(A) contacting the starting biological or other raw material containing one or all of the blood coagulation factors II, VII, IX and X with an ion exchanger sorbent on the basis of poly(diethylaminoethyl-hydroxyethylacrylate) and/or poly(diethylaminoethyl-hydroxyethylmethacrylate), equilibrated with a buffer solution of pH 7.2 to 7.6 having a saline concentration of 0.1 mol/l,

(B) removing the undesired accompanying proteins by washing with a buffer solution of pH 7.2 to 7.6 having a saline concentration of 0.2 mol/l,

and

(C) desorption of the coagulation factors II, VII, IX and/or X with a buffer solution of pH 7.2 to 7.6 having a saline concentration of 0.3 to 2.0 mol/l.

Depending on the desorption conditions in step C, functionally pure or mixtures of factors II, VII, IX and X are obtainable. Desorption is preferably carried out with use of the starting buffer, and by applying a saline gradient.

Synthetic sorbents based on a matrix of poly(DEAE-hydroxyethylmethacrylate) or poly(DEAE-hydroxyethylacrylate) particles are already known and commercially available products (cf. e.g. Separon, an ion exchanger on the basis of poly(DEAE-hydroxyethylmethacrylate) (poly(DEAE-HEMA) ), Tessek, Prague). These sorbents do not have the above described disadvantages of polysaccharide sorbents. The synthetic matrix is resistant to microbial contamination and does not change its quality during a change of physical or chemical conditions.

Because of the different matrix of the poly(hydroxyalkyl(meth)acrylate) sorbent as compared with polysaccharide ion exchanger sorbents, also the interaction between the coagulation factors II, VII, IX and X

during chromatographic separation on these materials is different to a certain degree.

The sorbents used according to the present invention are very stable and can be used for a long time without a change of their sorption properties.

In certain cases it may be useful to add proteinase inhibitors (e.g. benzamidine) for the blocking of auto-activation. The separation can be done in a chromatography (column) arrangement or using the batch method. The choice of the sorbent particle size depends on the applied separation technology. The advantageous physical and chemical properties of these sorbents lead to a considerable improvement of the complex fractionation of blood plasma to the required products.

Furthermore, the resistance of the sorbents against high pressure allows rapid and standard working cycles with the possibility of a precise reproduction of individual technological steps and an automation of the separation process. The character of the carrier allows a liquid flow under pressure, to keep the time limits of technological operations, and an improvement of their course. If the coagulation factors are prepared as more or less purified concentrates, they may be, after stabilization, pasteurized, e.g. for inactivation of viruses.

The method of the present invention can be used not only for separating and concentrating mixtures of the coagulation factors II, VII, IX and/or X, but also for the fractionation and isolation of these coagulation factors as such by appropriately selecting the desorption conditions in step c.

In accordance therewith, blood coagulation factor IX may be obtained by desorption in step C with a buffer solution of pH 7.2 to 7.6 having a saline concentration of 0.3 to 0.4 mol/l, whereas a mixture of coagulation factors II + X is obtained by desorption with a buffer solution of pH 7.2 to 7.6 having a saline concentration of 0.45 to 0.55 mol/l, and coagulation factor VII is obtainable by desorption in step C with a buffer solution of pH 7.2 to 7.6 having a saline concentration of 0.7 to 0.8 mol/l.

The control of the identity of the separated coagulation factors can be carried out with functional, i.e. coagulation, enzyme and immunochemical tests.

According to a preferred embodiment, the sorbent is used in a particulate, and preferably spherical, form and having a particle size of 50 to 500 µm and preferably of 80 to 100 µm.

The content of diethylaminoethyl groups present in the sorbent is preferably 0.5 to 5.0 mmol/g and more preferably 1.2 to 1.3 mmol/g.

As the buffer, a citrate buffer (preferably trisodium citrate), sodium acetate and/or Tris buffer are suited. For the saline content, sodium chloride is preferably used.

The starting material can be of human or animal origin and is preferably native plasma or a plasma concentrate, such as K-plasma.

The method of the present invention for preparing concentrates of coagulation factors II, VII, IX and X leads to considerable concentration (enrichment) factors of at least 20 to 30 over the normal starting plasma, i.e. leads to an at least 20- to 30-fold higher concentration of these factors than that in plasma.

In accordance therewith, also the specific activities (specific activity = activity (nkat/ml)/protein content (mg/ml) of the concentrates as obtainable according to the invention are considerably higher than those in the respective plasma: In the case of factor IX concentrates, the specific activities are at least 40 to 45 times that in plasma; the concentrates of factors II + X have specific activities of at least 50 to 65 times that in plasma, whereas the factor VII concentrates may have specific activities of > 70 times that in plasma. As regards the prothrombin concentrates of the present invention, the specific activity reaches values of > 100 times that in plasma.

The regeneration of the sorbent can be easily carried out by washing the sorbent with a buffer solution of pH 7.2 to 7.6 having a saline concentration of 2.0 mol/l, followed by distilled water and subsequent equilibration with buffer solution.

Another aspect of the present invention is based on the surprising observation, that the concentrates of coagulation factors II and X prepared according to the invention can be used for the detection and diagnosis of human and animal inflammatory diseases by means of qualitative detection or quantitative determination of coagulation factor VII.

Functionally pure coagulation factors VII, II and X are extensively used in diagnosis. The concentrates of factor II and X can be used to improve the quantitative determination of factor VII and some other factors in blood plasma and body fluids.

It is known, that an inflammatory process taking place in a mammalian organ is accompanied by an increase of permeability of blood vessel walls, whereby biologically active peptides (e.g. the kinins) are involved. In connection with the increased permeability ob blood vessel walls there appear in body fluids both corpuscular and protein components of the blood. On this basis are designed some indirect detection methods of uro-genital and glandula lactica inflammation, e.g. the detection of proteins and leukocytes in urine, the detection of albumin, $alpha_1$-antitrypsin and the so-called somatic cells in milk. These methods,

however, have some limitations regarding sensitivity and the necessary laboratory technology.

The importance of an early diagnosis of inflammatory diseases is widely accepted. Mastitis (inflammation of the glandula lactica), occuring in cattle, is an important barrier to constant high production and quality of milk. The later the illness is ascertained in the animal, the more difficult, long and expensive is the necessary treatment. The possibility of an early, specific and, as much as possible, simple and sensitive detection of mastitis incidence, mostly under the conditions of intensive husbandry, is very important. A high concentration of plasma proteins in milk also appears under physiological conditions during and after delivery (up to the $20^{th}$ day after delivery). When there appears a destabilization of vessel walls in milk-producing organs in a time not related to this period, it is always a signal of pathological processes. Quantitatively, the rate of protein penetration is proportional to the extent of mastitis; from the qualitative point of view proteins with a low molecular mass penetrate more easily. Detection of mastitis on the basis of determination of serum albumin or alpha$_1$-antitrypsin (Sandholm M. et al., J. Dairy Res. 51 - (1984) 1-9) in milk has been published. Albumin is difficult to detect by chromogenic tests; alpha$_1$-antitrypsin can be detected using chromogenic substrates and trypsin, but the process has certain methodological problems.

It has now been found in accordance with this aspect of the invention that plasma procoagulant (coagulation factor VII) appears very early in the course of kidney and glandula lactica inflammation in urine and milk, respectively.

On the basis of this observation, a method useful for the detection of inflammation of mammalian organs and the determination of its extent without the need for any prior pre-treatment of the respective body fluids has been achieved. The method may be generally used in all cases when body fluids, including diagnostic lavages, come into contact with the afflicted organ. The invention deals also with the detection or determination of the extent of an inflammatory disease using techniques of body fluid analysis in human and animal urine and milk, especially bovine milk.

The method according to the invention is based on the principle that a sample of the investigated biological material, e.g. body fluid is brought, either in one or in two steps, into contact with a solution containing thromboplastin, phospholipids and $Ca^{2+}$-ions and another solution, containing a concentrate of coagulation factors II and X, and afterwards, the liberated thrombin is detected with a chromogenic substrate, and the intensity of the resulting colour may be, if it is necessary, quantitatively determined, for example by comparison with a standard, or using an instrumental technology, e.g. colorimetry or spectrophotometry.

The method of this invention for the qualitative detection and the quantitative determination of blood coagulation factor VII in biological materials, particularly in human and animal plasma, plasma fractions, plasma preparations, milk or milk products, and body fluids, is characterized by

(a) contacting the sample of the biological material in one or two steps with a concentrate of blood coagulation factors II and X, particularly a concentrate according to the invention, in the presence of thromboplastin, phospholipids, $Ca^{2+}$-ions and a chromogenic substrate, and eventually coagulation factor V, and

(b) qualitatively detecting or quantitatively determining the resulting colour generated by the liberated thrombin and the chromogenic substrate.

This method is based on the finding that the plasma procoagulant, i.e. coagulation factor VII, is one of the first plasma proteins, which during inflammation of organs penetrate from the blood flow into the body fluids such as urine, milk etc. The concentration of this procoagulant in the body fluid is in direct proportion to the intensity of the ongoing inflammation and its size. Factor VII, together with tissue thromboplastin, $Ca^{2+}$-ions and phospholipids, initiates the so-called extrinsic pathway of blood coagulation activation:

Factor VII (plasma, milk, urine)

↓

Factor VIIa

Factor X ⟶ Factor Xa

↓ Factor V, phospholipids, $Ca^{2+}$

Factor II ⟶ Factor IIa (thrombin)

(prothrombin)

(Factors VIIa, Xa and IIa being the active forms of proenzymes).

Factor VII is activated by the action of a reagent, which contains tissue thromboplastin, $Ca^{2+}$, and, if necessary, phospholipids (reagent 1) to factor VIIa. In the case of milk, e.g., evidently activation components, i.e. $Ca^2$ and phospholipids, are already present. Upon addition (at once or stepwise) of the reagent containing a concentrate of factors II and X (reagent 2), factor X is gradually activated to factor Xa. Together with a cofactor (factor V), which is partially present in reagent 2 and partially also in body fluids of objects with a disequilibrated system of organ vessel walls, it converts factor II into factor IIa (thrombin), i.e. the proteinase, which under certain conditions splits suitable peptidic chromogenic substrates. Their cleavage products have an expressive and characteristic colour, on which may be based a sensitive detection method, and, by means of usual devices, also a quantitative determination.

Advantageous examples of such chromogenic substrates are peptidic derivatives of the formula
Z-Gly-Pro-Arg-X,
wherein
Z is a protective group, preferably benzyloxycarbonyl,
and
X is a chromophore derived from p-nitroaniline or is a N,N′-bis derivative of p-phenylene diamine bound by an azomethine bond to a chromophore aldehyde, preferably

4-dimethylaminobenzaldehyde

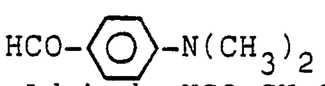 or 4-dimethylaminocinnam-

aldehyde 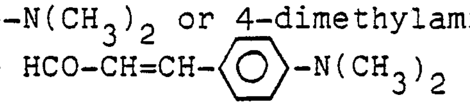 .

Accordingly, the thrombin-catalyzed reaction of this compound is as follows:
thrombin + Z-Gly-Pro-Arg-X→Z-Gly-Pro-Arg-OH + p-nitroaniline or

+ 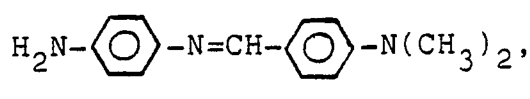,

whereby Z and X are as defined above.

The chromophore X is chosen according to the method of determination or the instrumental equipment, or depending on whether the detection is made in solution, or using a suitable diagnostic device (dry chemistry) on the basis of inert organic or inorganic materials which are generally known from laboratory practice. It is also possible to use substrates of another type, as long as they have suitable qualities.

The use of the factor II and X concentrate makes this method highly sensitive. Values obtained by instrumental measurement or by comparison with a colour standard give information about the extent of the

disease, and it may be said that the level of factor VII is minimal in body fluids of healthy subjects and high in subjects with organ inflammation. From the quantitative point of view, the level of factor VII is in good relation with the clinical status of the patient, and, due to the high sensitivity of the test, even a subclinical state of illness may be detected by this method, too.

The alternative of using a concentrate of factor X and the synthetic substrate for factor Xa instead of a concentrate of factors II and X is possible, however, the sensitivity of this alternative method does not reach the sensitivity of determination of factor VII through prothrombin/thrombin conversion. When the necessary instrumentation is available, namely autoanalyzers, then the quantitative spectrophotometric determination in large sets of samples is possible.

The use of diagnostic devices, for example diagnostics strips, is suitable for a quick semiquantitative test without great demand on laboratory or instrumental technique and personal qualification.

The method according to the invention is highly sensitive and specific, so that e.g. mastitis may be diagnosed even before the onset of clinical symptoms. Therefore, it is possible to begin the prophylactic treatment earlier, and the duration of the disease is shorter, and therefore exist also greater chances for a quick recovery. This way it is possible to prevent large economical losses. The most simple practical way of doing the test according to the invention is usually to put a sample, e.g. of milk, into sodium citrate (3.8 %, 1+9), and to analyze it immediately after transport to a laboratory having the necessary equipment. The samples may be stored in frozen state below -10 °C and, after accumulation of a number of samples, jointly analyzed after thawing. For milk it is also suitable to use absorptive material such as paper for absorbing the sample, to dry the sample and to analyze it later after elution. The last mentioned manner of sample preparation allows an easy transport of the samples into a central laboratory even from distant localities. The sample is then extracted for a certain time and at the same time activated using a first reagent comprising a calcium salt, thromboplastin and a buffer, whereby the following procedures are the same as for the analysis of native milk. Using this method, casein, the lipid components and cellular components remain on the absorption material, and the eluate containing factor VII is a clear liquid.

In accordance therewith, the method of this invention can advantageously be carried out by using a sample in step a which has been absorbed in or sorbed on a suitable carrier having a high absorptive capacity, preferably paper, and dried, and the charged dry carrier is extracted with simultaneous activation of factor VII with a buffered solution comprising a calcium salt and thromboplastin, and the activated extract is then contacted with the factors II and X and the chromogenic substrate under buffer conditions.

Alternatively, in step a, a liquid sample is contacted with a dry micture of a calcium salt, thromboplastin and a buffer and incubated, preferably at room temperature or at an elevated temperature ≤ 37 °C, and then the resulting mixture is brought into contact with a test strip comprising coagulation factors II and X, the chromogenic substrate and a buffer.

The analytical and diagnostic test kits for the qualitative detection or the quantitative determination of coagulation factor VII in biological samples, comprise the following components:

(I)

(A) a first reagent comprising a calcium salt, thromboplastin and a buffer,

(B) a second reagent comprising the coagulation factors II and X and a buffer,

and

(C) a third reagent comprising a chromogenic substrate;

or

(II)

(A) a first reagent comprising a calcium salt, thromboplastin and a buffer,

and

(B) a second reagent comprising the coagulation factors II and X, a chromogenic substrate and a buffer,

or

(III) one single, combined reagent comprising a calcium salt, thromboplastin, the coagulation factors II and X, a chromogenic substrate and a buffer.

If necessary, the first and/or the second reagent or the single, combined reagent comprise a phospholipid and eventually, depending on the kind of chromogenic substrate used, an acidification agent.

In the following, the invention will be explained with reference to practical examples.

## Example 1

### Preparation of Concentrate of Coagulation Factors II, VII, IX and X

As a source of the coagulation factors normal human plasma (native fresh plasma) or the so-called K-plasma (plasma free of cryoprecipitate) are used, with a controlled pH of 7.2 - 7.6. 1000 ml of plasma were brought into contact with poly(DEAE-hydroxyethylmethacrylate) gel in a column (100-150 ml) equilibrated with the starting buffer of pH 7.4 (0.01 mol/l trisodium citrate containing 0.1 mol/l sodium chloride). The size of the sorbent particles was 80 to 100 µm. After the sorption of coagulation factors non-sorbed and weakly bound proteins were removed by washing with the starting buffer containing 0.2 mol/l sodium chloride. The desorption of all other proteins, which after washing remain bound on the sorbent, was done with increasing concentrations of saline in the starting buffer (up to 2.0 mol/l). The obtained eluate did contain all factors of the prothrombin complex, i.e. factors II, VII, IX, X, and protein C.

The fraction can then be processed into a solution suitable for substitution therapy. Using a sorbent containing 1.2 to 1.3 mmol/g of DEAE-groups and having a particle size of 80 to 100 µm, it is possible to separate with the described procedure from normal human plasma, 60 to 90 % of its content of the prothrombin complex components. These proteins represent the main protein component of the obtained concentrate.

The obtained concentrate of coagulation factors II, VII, IX and X had a purification (enrichment) factor of 20 to 30 over plasma, i.e. a 20- to 30-fold higher concentration than that in plasma.

Regeneration of the sorbent is performed by intensive washing of the sorbent with citrate buffer, containing 2.0 mol/l sodium chloride, followed by distilled water and starting buffer for the next working cycle.

## Example 2

### Preparation of Concentrate of Factors II and X, besides Factor IX and Factor VII

1000 ml of human plasma were passed through a column of poly(DEAE-hydroxyethylmethacrylate) under the same conditions as in Example 1. After removal of undesired proteins by citrate buffer of pH 7.4, containing 0.2 mol/l of saline, elution was carried out with a salt gradient from 0.2 to 0.8 mol/l saline in the starting buffer of pH 7.4. The absorbancy of the eluted proteins was measured with a registering photometer at 280 nm. The fraction containing factor IX was eluted from the column at a saline concentration of 0.3 to 0.4 mol/l; a solution containing factors II and X was obtained at a saline concentration of 0.45 to 0.55 mol/l, and at a saline concentration of 0.7 to 0.8 mol/l, a fraction containing functionally pure factor VII was eluted from the column.

For some purposes it is suitable to pool fractions containing factor IX and factors II + X into one preparation. At a saline concentration of 0.5 mol/l a fraction containing factors IX, II and X, was obtained, after increase of the NaCl concentration to 0.8 mol/l a fraction was eluted with localized factor VII.

In all cases the process took place in the starting buffer of pH 7.4. The obtained concentrate of factor VII is free of other factors of the prothrombin complex, and the concentrate of factors IX, II and X is free of factor VII. The quantitative ratios are similar to those of Example 1.

The obtained factor IX concentrate had a specific activity of 40 to 45 times that in plasma; the specific activity of the concentrate of factors II + X was 50 to 65 times that in plasma, whereas the specific activity of the factor VII concentrate was more than 70 times that in plasma.

## Example 3

### Preparation of Prothrombin Concentrate (Factor II) from the washing solution (0.4 mol/l sodium acetate, pH 7.4) obtained at the separation of Plasma Antithrombin III

The fraction obtained by separation of human antithrombin III on a heparin-poly-(hydroxyethylmethacrylate) sorbent contains, in addition to a little amount of factors IX, X and protein C, a

considerable amount of prothrombin, which can be separated by the method according to the invention.

1000 ml of the washing solution were diluted with 1000 ml of distilled water and filtered through a column of poly(DEAE-hydroxyethylmethacrylate) which was equilibrated with 0.2 mol/l sodium acetate at pH 7.4. After sorption and washing the column with an acetate solution (0.2 mol/l) desorption was carried out with saline in buffer in the same manner as in Example 1.

The specific activity of the obtained prothrombin concentrate was 80 to 100 times that in human plasma.

## Example 4

### Preparation of Prothrombin Concentrate from Ethanol Fraction III

As a raw material for the preparation of prothrombin an ethanol fraction III, was used, obtained during fractionation of human blood plasma.

100 g of this fraction in the form of a wet precipitate was suspended in 1 liter of citrate buffer of pH 7.4 (0.01 mol/l with 0.1 mol/l saline). After 2 hours mixing at 0 to 4 °C the suspension was centrifugated, and the supernatant was processed in the same way as the plasma in Examples 1 or 2.

Before the final processing, the supernatant can be filtered, if necessary.

The specific activity of the obtained prothrombin concentrate was more than 100 times that in plasma.

## Example 5

### Preparation of "Prothrombin Complex" Coagulation Factors from Bovine Plasma

The separation was performed according to Example 2 with the exception that as the starting material bovine blood plasma was used. The separated concentrate of factors II and X was used as a reagent for the identification and determination of factor VII in milk, and accordingly, the detection of bovine mastitis.

The specific activities of the obtained concentrates were the same as in Example 2.

## Example 6a

### Determination of Coagulation Factor VII in Cattle Milk

A sample of milk (0.1 to 10 µl) is mixed with 200 µl of reagents 1 + 2 (the preparation of which is explained below) and incubated for 5 min at 37 °C, or for 10 min at 25 °C. Then the chromogenic substrate (e.g. Z-Gly-Pro-Arg-p-nitroanilide, concentration 0.3 mol/l) is added to the mixture, and the rate of hydrolysis is determined with a spectrophotometer as the amount of split-off p-nitroaniline ($\Delta A_{405}$/min). On the basis of the amount of liberated p-nitroaniline the activity of thrombin and the concentration of factor VII in milk can be calculated.

### Combined reagents 1 + 2:

5 ml of concentrate of factors II and X in Tris buffer pH 8.2 (0.05 mol/l) are mixed with a solution (5 ml) of thromboplastin, stored at a temperature below -40 °C and used after thawing. A lyophilised concentrate of factors II and X may be also mixed with lyophilised thromboplastin, homogenized and tableted. Using the combined reagent 1 + 2 a sample of milk is diluted (1 + 100) with Tris buffer of pH 8.2 containing $CaCl_2$ - (0.005 mol/l).

## Example 6b

A sample of milk (0.1 to 10 µl) is mixed with 200 µl of reagent 1 and 50 µl of reagent 2 (the preparation of which is explained below) and incubated for 5 min at 37 °C, or for 10 min at 25 °C. Subsequently, the substrate is added to the mixture which is then processed as in Example 6a.

**Reagent 1:**

The reagent is prepared by mixing 2 ml of Tris buffer (0.05 mol/l) of pH 8.2, containing 0.005 mol/l of calcium chloride and 0.5 ml of a thromboplastin solution (in such a concentration that 200 µl of the resulting mixture activate the amount of factor VII present in 1 µl of normal human or bovine plasma). For the determination of factor VII in 0.1 to 10 µl of milk 200 µl of the above mentioned reagent are used.

**Reagent 2**

A concentrate of factors II and X is dissolved in Tris buffer of pH 8.2 (0.05 mol/l) and used for the determination of factor VII in 0.1 to 10 µl of milk in such a volume that it would contain about 10 to 20 nkat of factor II and 1 to 2 nkat of factor X per ml. The reagent must be quite devoid of factor VII.

**Example 7**

The procedure of Example 6b is followed with the exception that the sample of milk is absorbed in a material with a high absorptive capacity (paper) and dried. Factor VII is extracted before the analysis for 20 - 60 min with simultaneous activation by reagent 1. The clear, activated eluate is analyzed with reagent 2. A paper area of 20 $mm^2$ is extracted with 200 to 400 µl of the solution of reagent 1. The composition of reagents 1 and 2 is the same as in Example 6b.

**Example 8**

A defined volume of milk (or pre-diluted milk) is put into a small bottle, containing reagent 1 in a suitable form (tableted, lyophilised) in such an amount that it would complete the activation of factor VII present in the milk in 10 min at 25 °C. After this time (which should not be longer than 1 hour) a diagnostic strip is dipped into the reagent mixture containing reagent 2, buffer of pH 8.2 and the chromogenic substrate (for example on the basis of chromophore arylaldehyde such as Z-Gly-Pro-Arg-NH-$C_6$-$H_4$-N=CH-$C_6H_4$-N$(CH_3)_2$, or e.g. with 4-dimethylaminocinnamaldehyde, 50 µl of 2 mmol/l solution per $cm^2$). The product of the thrombin-catalyzed hydrolysis gives after acidification (reaction with chromophore) an intensive coloured substance (blue). The intensity of the colour is directly proportional to the quantity of factor VII in the milk. The evaluation is made either semiquantitatively by comparison with a coloured standard, or quantitatively by measurement of the intensity of the colour with a photometer (e.g. a reflexion photometer). The composition of reagents 1 and 2 is the same as in Example 6b.

**Example 9**

The procedure of Example 8, is followed with the exception that the strip contains, besides the substrate, buffer, chromophore and acidification agent, also reagents 1 + 2. The composition is the same as in Example 6a.

**Example 10**

The procedure of Examples 6, 8 or 9 is followed with the exception that human urine or another body fluid, eventually a solution used for washing the afflicted organ is used as the sample. In healthy subjects, the level of factor VII in urine is minimal, and an increase is an indication of an inflammatory process of the urogenital tract, eventually of the organ, where the lavage was made.

## Claims

1. Concentrates of blood coagulation factors II, VII, IX and/or X, obtainable by contacting a biological material containing these factors with a sorbent on the basis of poly(diethylaminoethyl-hydroxyethylacrylate) and/or poly(diethylaminoethyl-hydroxyethylmethacrylate) and desorption with a buffer solution of pH 7.2 to 7.6 having a saline concentration of 0.3 to 2.0 mol/l.

2. Concentrates according to claim 1 obtainable by

(A) contacting a biological material containing one or all of the blood coagulation factors II, VII, IX and X with a sorbent on the basis of poly(diethylaminoethyl-hydroxyethylacrylate) and/or poly(diethylaminoethyl-hydroxyethylmethacrylate), equilibrated with a buffer solution of pH 7.2 to 7.6 having a saline concentration of 0.1 mol/l,

(B) removing the undesired accompanying proteins by washing with a buffer solution of pH 7.2 to 7.6 having a saline concentration of 0.2 mol/l,

and

(C) desorption of the coagulation factors II, VII, IX and/or X with a buffer solution of pH 7.2 to 7.6 having a saline concentration of 0.3 to 2.0 mol/l.

3. Concentrates of blood coagulation factor IX according to claim 2, obtainable by desorption in step C with a buffer solution of pH 7.2 to 7.6 having a saline concentration of 0.3 to 0.4 mol/l.

4. Concentrates of blood coagulation factors II + X according to claim 2, obtainable by desorption in step C with a buffer solution of pH 7.2 to 7.6 having a saline concentration of 0.45 to 0.55 mol/l.

5. Concentrates of blood coagulation factor VII according to claim 2, obtainable by desorption in step C with a buffer solution of pH 7.2 to 7.6 having a saline concentration of 0.7 to 0.8 mol/l.

6. Concentrates of blood coagulation factors II, VII, IX and/or X according to one of claims 1 to 5, characterized in that the coagulation factors are human factors.

7. A method of preparing concentrates of blood coagulation factors II, VII, IX and/or X according to one of claims 1 to 6, characterized by contacting a biological material containing these factors with a sorbent on the basis of poly(diethylaminoethyl-hydroxyethylacrylate) and/or poly(diethylaminoethyl-hydroxyethylmethacrylate) and desorption with a buffer solution of pH 7.2 to 7.6 having a saline concentration of 0.3 to 2.0 mol/l.

8. The method according to claim 7, characterized by the following steps:

(A) Contacting a biological material containing one or all of the blood coagulation factors II, VII, IX and X with a sorbent on the basis of poly(diethylaminoethyl-hydroxyethylacrylate) and/or poly(diethylaminoethyl-hydroxyethylmethacrylate), equilibrated with a buffer solution of pH 7.2 to 7.6 having a saline concentration of 0.1 mol/l,

(B) removing the undesired accompanying proteins by washing with a buffer solution of pH 7.2 to 7.6 having a saline concentration of 0.2 mol/l,

and

(C) desorption of the coagulation factors II, VII, IX and/or X with a buffer solution of pH 7.2 to 7.6 having a saline concentration of 0.3 to 2.0 mol/l.

9. The method according to claim 8 for preparing concentrates of blood coagulation factor IX, characterized by desorption in step C with a buffer solution of pH 7.2 to 7.6 having a saline concentration of 0.3 to 0.4 mol/l.

10. The method according to claim 8 for preparing concentrates of blood coagulation factors II + X, characterized by desorption in step C with a buffer solution of pH 7.2 to 7.6 having a saline concentration of 0.45 to 0.55 mol/l.

11. The method according to claim 8 for preparing concentrates of blood coagulation factor VII, characterized by desorption in step C with a buffer solution of pH 7.2 to 7.6 having a saline concentration of 0.7 to 0.8 mol/l.

12. The method according to one of claims 7 to 11, characterized in that a particulate sorbent is used having a particle size of 50 to 500 µm and preferably of 80 to 100 µm.

13. The method according to one of claims 7 to 12, characterized in that a sorbent is used having a content of diethylaminoethyl groups of 0.5 to 5 mmol/g and preferably of 1.2 to 1.3 mmol/g.

14. The method according to one of claims 7 to 13, characterized in that the process is carried out in a chromatographic column or according to a batch method.

15. The method according to one of claims 7 to 14, characterized in that citrate buffer solutions containing trisodium citrate and sodium chloride are used.

16. The method according to one of claims 7 to 15, characterized in that native plasma or cryoprecipitate-free plasma (K-plasma) is used as starting material.

17. The method according to one of claims 7 to 16, characterized in that a human starting material is used.

18. Use of concentrates of blood coagulation factors II and X, particularly of those according to one of claims 1, 2, 4 and 6, for the qualitative detection and the quantitative determination of blood coagulation factor VII in biological materials.

19. A method for the qualitative detection and the quantitative determination of blood coagulation factor VII in biological materials, particularly in human and animal plasma, plasma fractions, plasma preparations, milk or milk products, and body fluids,
characterized by

(a) contacting the sample of the biological material in one or two steps with a concentrate of blood coagulation factors II and X, particularly a concentrate according to one of claims 1, 2, 4 and 6, in the presence of thromboplastin, phospholipids, $Ca^{2+}$ ions and a chromogenic substrate, and eventually coagulation factor V,
and

(b) qualitatively detecting or quantitatively determining the resulting colour generated by the liberated thrombin and the chromogenic substrate.

20. The method according to claim 19, characterized in that in step a the sample is brought into contact with the reagents in two subsequent steps.

21. The method according to claim 19 or 20, characterized in that in step a the sample is first brought into contact with a calcium salt and thromboplastin under buffer conditions and the resulting liquid is then contacted with the factors II and X, the chromogenic substrate and a buffer.

22. The method according to claim 19 or 20, characterized in that in step a the sample is first brought into contact with a calcium salt, thromboplastin and the factors II and X under buffer conditions, and the resulting liquid is then contacted with the chromogenic substrate.

23. The method according to claim 19 or 20, characterized in that in step a, a sample is used which has been absorbed in or sorbed on a suitable carrier having a high absorptive capacity, preferably paper, and dried, and the charged dry carrier is extracted with simultaneous activation of factor VII with a buffered solution comprising a calcium salt and thromboplastin, and the activated extract is then contacted with the factors II and X and the chromogenic substrate under buffer conditions.

24. The method according to one of claims 19 to 22, characterized in that in step a, a liquid sample is contacted with a dry micture of a calcium salt, thromboplastin and a buffer and incubated, preferably at room temperature or at an elevated temperature $\leq 37\ ^{\circ}C$, and then the resulting mixture is brought into contact with a test strip comprising coagulation factors II and X, the chromogenic substrate and a buffer.

25. The method according to one of claims 19 to 24, characterized in that as chromogenic substrate, a peptidic compound of the formula
Z-Gly-Pro-Arg-X
is used,
wherein
Z is a protective group, preferably benzyloxycarbonyl,
and
X is a chromophore derived from p-nitroaniline or a N,N′-bis derivative of p-phenylene diamine bound by an azomethine bond to a chromophore aldehyde, preferably 4-dimethylaminobenzaldehyde or 4-dimethylaminocinnamaldehyde.

26. Analytical and diagnostic kits for the qualitative detection or the quantitative determination of coagulation factor VII in biological samples, particularly for carrying out the method according to one of claims 19 to 25,
characterized in that they comprise the following components:

(I)
(A) a first reagent comprising a calcium salt, thromboplastin and a buffer,
(B) a second reagent comprising the coagulation factors II and X and a buffer,
and
(C) a third reagent comprising a chromogenic subtrate;
or

(II)

(A) a first reagent comprising a calcium salt, thromboplastin and a buffer,

and

(B) a second reagent comprising the coagulation factors II and X, a chromogenic substrate and a buffer,

or

(III) one single, combined reagent comprising a calcium salt, thromboplastin, the coagulation factors II and X, a chromogenic substrate and a buffer.

27. The analytical and diagnostic kits according to claim 26, characterized in that the first reagent and/or the second reagent are present in a dry state.

28. The analytical and diagnostic kits according to claim 26 or 27, characterized in that the first reagent and/or the second reagent or the single, combined reagent are present in the form of dry strips.

29. The analytical and diagnostic kits according to one of claims 26 to 28, characterized in that the first and/or the second reagent or the single, combined reagent comprise a phospholiquid and/or an acidification agent.